# EUROPEAN PATENT SPECIFICATION

(11) **EP 4 147 242 B1**
(45) Date of publication and mention of the grant of the patent: **12.11.2025**
(21) Application number: 21729693.8
(22) Date of filing: 07.05.2021
(51) Int. Cl.: G16B 30/10, G16B 20/20, G16H 50/80

(54) **GENOME SEQUENCING AND DETECTION TECHNIQUES**
GENOMSEQUENZIERUNGS- UND -DETEKTIONSTECHNIKEN
TECHNIQUES DE SÉQUENÇAGE ET DE DÉTECTION DE GÉNOME

(30) Priority: 08.05.2020 US 202063022296 P
(43) Date of publication of application: 15.03.2023
(73) Proprietor: Illumina, Inc., San Diego, CA 92122 (US)
(72) Inventor: BILKE, Sven, San Diego, California 92122 (US); SCHLESINGER, Johann Felix Wilhelm, San Diego, California 92122 (US)
(74) Representative: Robinson, David Edward Ashdown
(86) International application number: PCT/US2021/031390
(87) International publication number: WO 2021/226523

(56) References cited:
- WO-A1-2015/083004
- WINGETT STEVEN W ET AL: "FastQ Screen: A tool for multi-genome mapping and quality control", F1000 RESEARCH, 24 August 2018 (2018-08-24), pages 1 - 13, XP055833709, Retrieved from the Internet <URL:https://www.ncbi.nlm.nih.gov/pmc/articles/PMC6124377/pdf/f1000research-7-17815.pdf> [retrieved on 20210820], DOI: 10.12688/f1000research.15931.2
- BABRAHAMBIOINF: "FastQ Screen Filtering Tutorial", YOUTUBE, 15 June 2018 (2018-06-15), pages 1, XP054982156, Retrieved from the Internet <URL:https://www.youtube.com/watch?v=eJcAv-Dt57I&list=PLbiByRpDb_hO2dFQDTYeyx4i6X10UtIXG&index=5> [retrieved on 20210823]
- BABRAHAMBIOINF: "Interpreting_FastQ_Screen_Results", YOUTUBE, 25 October 2016 (2016-10-25), pages 1, XP054982155, Retrieved from the Internet <URL:https://www.youtube.com/watch?v=x32k84HHqjQ&list=PLbiByRpDb_hO2dFQDTYeyx4i6X10UtIXG&index=4> [retrieved on 20210823]
- ZHANG FAN ET AL: "FASTQuick: rapid and comprehensive quality assessment of raw sequence reads", OXFORD GIGASCIENCE, vol. 10, no. 2, 29 January 2021 (2021-01-29), pages 1 - 7, XP055833692, Retrieved from the Internet <URL:https://academic.oup.com/gigascience/article/10/2/giab004/6123620> DOI: 10.1093/gigascience/giab004
- BOLGER ANTHONY M. ET AL: "Trimmomatic: a flexible trimmer for Illumina sequence data", BIOINFORMATICS, vol. 30, no. 15, 1 April 2014 (2014-04-01), GB, pages 2114 - 2120, XP055862121, ISSN: 1367-4803, Retrieved from the Internet <URL:https://www.ncbi.nlm.nih.gov/pmc/articles/PMC4103590/pdf/btu170.pdf> DOI: 10.1093/bioinformatics/btu170
- SHIFU CHEN ET AL: "AfterQC: automatic filtering, trimming, error removing and quality control for fastq data", BMC BIOINFORMATICS, BIOMED CENTRAL LTD, LONDON, UK, vol. 18, no. 3, 14 March 2017 (2017-03-14), pages 91 - 100, XP021243183, DOI: 10.1186/S12859-017-1469-3

## Description

### BACKGROUND

The disclosed technology relates generally to nucleic acid characterization, e.g., sequencing techniques. In some embodiments, the technology disclosed includes fast, accurate methods for viral detection from sequence data based on genome sequencing, e.g., whole genome sequencing.

Next generation sequencing technology is providing increasingly high speed of sequencing, allowing larger sequencing depth. However, sequencing accuracy and sensitivity are affected by errors and noise from various sources, e.g., sample defects or PCR bias during library preparation. Therefore, detection of sequences of very low frequency, such as in a host sample that includes a low concentration of viral or bacterial nucleic acid, may be complex. Therefore, it is desirable to develop methods for detecting and/or sequencing nucleic acid molecules present in low quantities in a fast and accurate manner.

In the article by Wingett Steven W. et al.: "FastQ Screen: A tool for multi-genome mapping and quality control",F1000 Research, 24 August 2018, DOI: 10.12688/f1000research.15931.2, URL:https://www.ncbi.nlm.nih.gov/pmc/articles/PMC6124377/pdf/f1000research-7-17815.pdf , multi-genome mapping and quality control is disclosed: FastQ Screen is a tool to validate the origin of DNA samples by quantifying the proportion of reads that map to a panel of reference genomes. FastQ Screen is intended to be used routinely as a quality control measure and for analysing samples in which the origin of the DNA is uncertain or has multiple sources.

Zhang Fan et al.: "FASTQuick: rapid and comprehensive quality assessment of raw sequence reads",OXFORD GIGASCIENCE,vol. 10, no. 2, 29 January 2021, DOI: 10.1093/gigascience/ giab004, URL:https://academic.oup.com/gigascience/article/10/2/giab004/6123620 , discloses rapid and accurate methods to produce comprehensive quality metrics directly from a subset of raw sequence reads without full alignment using k-mer hash tables, providing a plurality of quality metrics, including estimates of genetic ancestry and cross-sample contamination. The tool enables detection of potential quality issues in real-time.

### BRIEF DESCRIPTION

In one embodiment, the present disclosure relates to a real-time quality control method. The method includes generating sequence data from a biological sample using a sequencing device conducting a sequencing run; identifying k-mers in the sequence data that have an exact match in a hash table that is initialized with a set of k-mers comprising a reference allele k-mer and an alternative allele k-mer of the reference allele; determining a distribution of the reference allele and the alternative allele in the sequence data based on a count of the exact matches of the reference allele k-mers relative to the alternative allele k-mers; and generating a passing or failing quality metric on the sequencing device for the biological sample based on the distribution and during the sequencing run of the biological sample.

In another embodiment, the present disclosure relates to a sequencing device that includes a substrate having loaded thereon a sequencing library prepared from a sample. The sequencing device also includes a computer programmed to cause the sequencing device to conduct a sequencing run to generate sequence data from sequencing library; identify k-mers in the sequence data that have an exact match in a hash table that is initialized with a set of k-mers comprising a reference allele k-mer and an alternative allele k-mer of the reference allele; determine a distribution of the reference allele and the alternative allele in the sequence data based on a count of the exact matches of the reference allel k-mers relative to the alternative allel k-mers; and generate a failing quality metric on the sequencing device for the biological sample based on the distribution being indicative of error or contamination during the sequencing run.

The scope of the technology disclosed is defined by the appended claims.

### BRIEF DESCRIPTION OF THE DRAWINGS

These and other features, aspects, and advantages of the present invention will become better understood when the following detailed description is read with reference to the accompanying drawings in which like characters represent like parts throughout the drawings, wherein:
FIG. 1 is a schematic illustration of a workflow for k-mer alignment, in accordance with aspects of the present disclosure;
FIG. 2 is a schematic illustration of example k-mers for a genome, in accordance with aspects of the present disclosure;
FIG. 3 is a schematic illustration of a method for viral detection from sequencing data, in accordance with aspects of the present disclosure;
FIG. 4 is a schematic illustration of a method for alignment-based viral detection, in accordance with aspects of the present disclosure;
FIG. 5 is a schematic illustration of a target region or k-mer coverage in alignment-based viral detection, in accordance with aspects of the present disclosure;
FIG. 6 is a schematic illustration of a method of generating a set of pathogen-specific k-mers and control k-mers for pathogen detection, in accordance with aspects of the present disclosure;
FIG. 7 is a block diagram of a system configured to acquire sequencing data and perform alignment-based detection, in accordance with aspects of the present disclosure;
FIG. 8 shows an example workflow for sample preparation for pathogen detection;
FIG. 9 shows amplicon sequencing results for the workflow of FIG. 8; and
FIG. 10 shows variant identification after primer trimming.

### DETAILED DESCRIPTION

The following discussion is presented to enable any person skilled in the art to make and use the technology disclosed, and is provided in the context of a particular application and its requirements.

Described herein are a variety of methods and compositions that allow for the characterization of nucleic acids. In an embodiment, the disclosed techniques are used as part of sequence analysis of sequence data generated from a biological sample to quickly and accurately detect genome sequences of interest. In an embodiment, the disclosed techniques use an ultra-fast hash-based aligner for generating reduced error or error-free sub-sequences from sequence data. One application of the disclosed techniques is rapid detection of viral genomes present in a sequenced library. The technique operates to scan each k-mer of fixed size "n" in every sequence read of a sequenced library and look up presence / absence in a hash-table. The hash-table is initialized with all n k-mers of the viral genome or a curated subset thereof. For example, curation can be used to remove k-mers that are not unique to the pathogen(s) of interest. Successful matches of a sequence k-mer against the hash-table are counted for each viral k-mer.

In embodiments, a specialized aligner using fast, exact k-mer matching of a full or reduced (e.g., curated) set of k-mers that are unique to the virus are used to detect pathogen infection with human positive control amplicons. However, the disclosed techniques may be used in other applications, such as detection of germline variants in a biological sample, microbiome characterization, detection of pooled or complex input samples in environmental monitoring (e.g., sewage monitoring). Further, the disclosed techniques may be used for detection of a single pathogen of interest (e.g., SARS-CoV-2) or detection of one or more pathogens in a pathogen panel, e.g., a respiratory pathogen panel (SARS-CoV-2, RSV, pneumonia, influenza), or a strain tracking panel including k-mers representative of different strains of a particular pathogen.

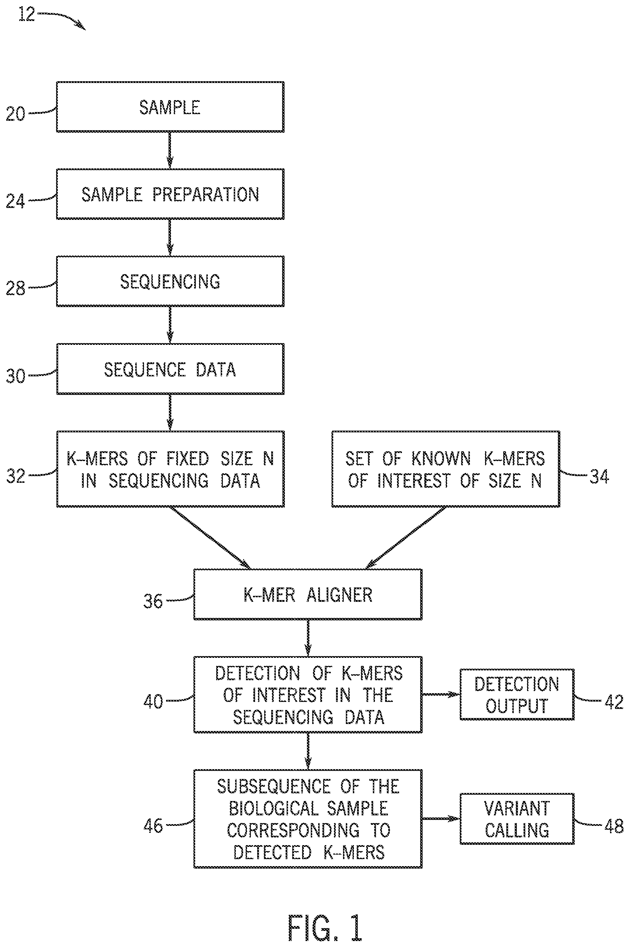

FIG. 1 is an example workflow 12 that includes steps of sample processing through sequence analysis that may be used in conjunction with the disclosed techniques. A sample 20 undergoes processing or sample preparation 24 to generate a sequencing library including a plurality of nucleic acid fragments suitable for sequencing steps 28 to generate sequence data 30. The sequence data 30 may undergo certain primary analysis steps, e.g., quality or filtering, before being passed to k-mer scanning and k-mer alignment as generally provided herein.

The generated sequence data 30 is scanned to identify k-mers of a fixed size n, and these identified k-mers are provided to a k-mer aligner 36. The k-mer aligner 36 may include a hash-table that is initialized with a set 34 of known k-mers of size n derived from a reference genome. The reference genome may be all the size n k-mers of interest of a pathogen genome (or a curated subset thereof) or other sequences of interest as provided herein.

The sequence data 30 may be streamed to the k-mer aligner 36 in real-time or on a rolling basis, such that the k-mer aligner 36 operates at block 40 on available additional sequence data 30 as it is received to detect k-mers of interest in the sequence data 30. The k-mer aligner 36 identifies k-mers in the sequence data 30 that are exact matches for the set of k-mers of interest 34. Exact matches may contribute to a total count of matches for the sample 20. Once the sample 20 passes a threshold count of identified k-mer exact matches, the workflow 12 provides a detection output 42. In an embodiment, an individual sample 20 can be characterized as positive or negative for detection of the sequences in the set 34. Because the k-mer aligner 36 operates on real-time streaming data, the detection functionality permits rapid identification of a status of the sample 20 using k-mer exact matches as soon as the threshold count is passed. Further, the k-mer based detection is less computationally intensive than conventional alignment-based techniques and, in embodiments, other k-mer techniques. In one example, the disclosed techniques use a fixed k-mer size n. Thus, the k-mer matching is based on matching only k-mers of size n and not matching all k-mers of all possible sizes or within a range of k-mer sizes. In another example, within the set of all possible k-mers of the fixed size n, the technique assesses matching for only a known subset based on the known sequence of the reference genome.

The resulting k-mer counts for each sample 20 are used as provided herein to characterize the sample to provide the detection output 42, e.g., determining a pathogen infection status. For example, k-mer counts above a threshold are indicative of a positive result for the presence of the pathogen in the sample. A negative result is indicative of no or below a threshold levels of k-mer counts in the sample. The k-mer counts may be assessed relative to a global threshold reflective of a total k-mer match count per sample 20. In other embodiments and as disclosed herein, the k-mer counts may be assessed on a per-target region basis and/or may be subjected to quality metrics before contributing to the k-mer count and detection of the pathogen, e.g., a positive or negative result.

The detection output 42 may include, in embodiments, providing a notification, message, or report indicative of a characteristic of the sample 20, e.g., a positive detection result, a negative detection result. The detection output 42 may, in embodiments, control subsequent processing steps of the sequence data 30. In contrast to conventional alignment-based detection that passes all or most incoming data to secondary analysis, the workflow 12 may limit additional processing to a subset of samples that are positive for a pathogen or other genome/sequence of interest. That is, once identified, only positive samples 20 may be passed to additional or secondary sequence analysis. In this manner, the workflow 12 improves allocation of processing resources by not devoting resources to secondary analysis of samples that are likely not to include the sequences of interest based on k-mer matching. Additional sequence analysis may include determining subsequences of the biological sample at block 46 to generate a variant calling output 48. Thus, potentially time-consuming analysis, i.e., alignment to the reference genome and variant calling, can in this way be restricted to positive (e.g., infected) samples after identification. Further, samples 20 that are not yet identified as positive can continue to be assessed by the k-mer aligner 36 until sufficient data is acquired to confirm a negative or positive result. An additional benefit of the disclosed techniques is that the k-mer based detection happens in real-time and based on relatively rapid analysis. Therefore, the processing efficiency improvements are achieved without significant delay to initiating secondary analysis for the relevant subset of positive samples. Further, for some analysis runs, the workflow 12 may terminate after the detection output 42 without advancing to subsequent analysis or variant calling in block 46.

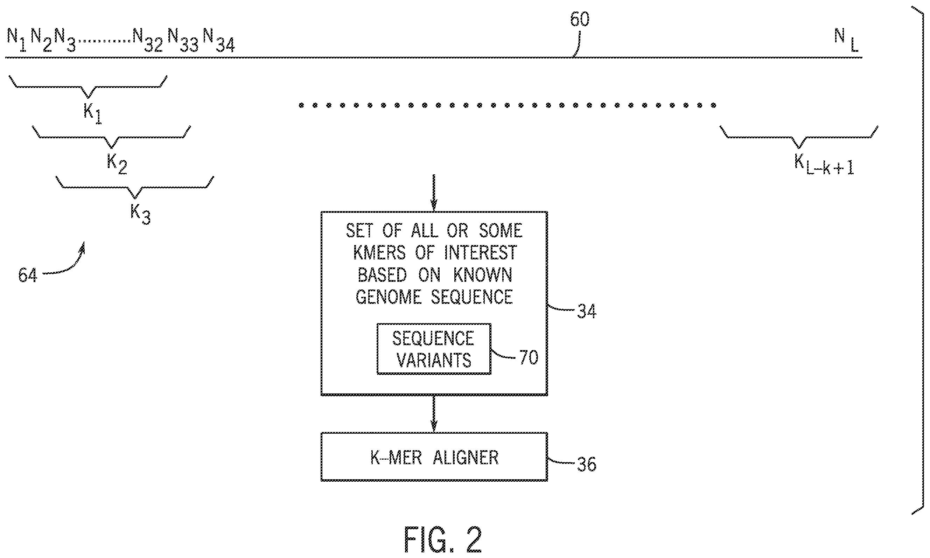

FIG. 2 is a schematic illustration of k-mers 64 of a nucleic acid 60 that form the set 34 of k-mers of interest of the k-mer aligner 36 (see FIG. 1). The nucleic acid 60 may be representative of all or part of a reference genome or previously characterized genome of interest, e.g., a pathogen genome. Thus, the disclosed techniques may be reference-free in the sense that the reference genome need not be sequenced together with the sample 20, and the set 34 may be computationally built based on stored or accessed reference sequence data of the nucleic acid 60. In embodiments, the nucleic acid 60 may be a reverse complement of and/or a cDNA copy of a single-stranded reference genome.

As provided herein, k-mer or k-mers refer to a contiguous substring or substrings of length "k" contained within a biological sequence such as a nucleic acid sequence. A set of k-mers may refer to all or only some subsequences contained within a nucleic acid of length L. A known or characterized sequence of length L will have total k-mers and an uncharacterized or unknown sequence can have x^{k} possible or potential k-mers, where x is the number of possible monomers (e.g., four in the case of DNA or RNA).

In an embodiment, k-mers are used at a fixed size n such that, for a given operation, all k-mers used for building the set of k-mers 34 and for scanning the sequence data are a same, fixed size relative to one another. However, different k-mers of a same size represent different sequence strings at different or shifted locations relative to one another. In certain embodiments, k-mers with length=32 (which can be efficiently analyzed on a 64bit CPU) are used for the k-mer matching, but any size k-mers with a fixed length greater than 24 could be used. Accordingly, the fixed k-mer length may be 25, 26, 27, 28, 29, 30, and so on.

While the nucleic acid 60 may include sequences that are previously characterized, additional sequences such as known or predicted variants 70 may be included. The disclosed reference-free techniques advantage of the fact that the variants in the viral genome are rare relative to the total size of the virus. During k-mer alignment, k-mers from the sample sequence data that include/overlap the variant would be 'lost' because they would fail to have exact matches in a hash table initialized with a variant-free set of reference k-mers 34. However, since variants are rare relative to the total size of the virus, this only leads to a minimal loss in sensitivity. In some methods, known variants present in the population also may be included as one or more 'variant k-mers' 34 added to the set of k-mers 34 in the k-mer aligner 36.

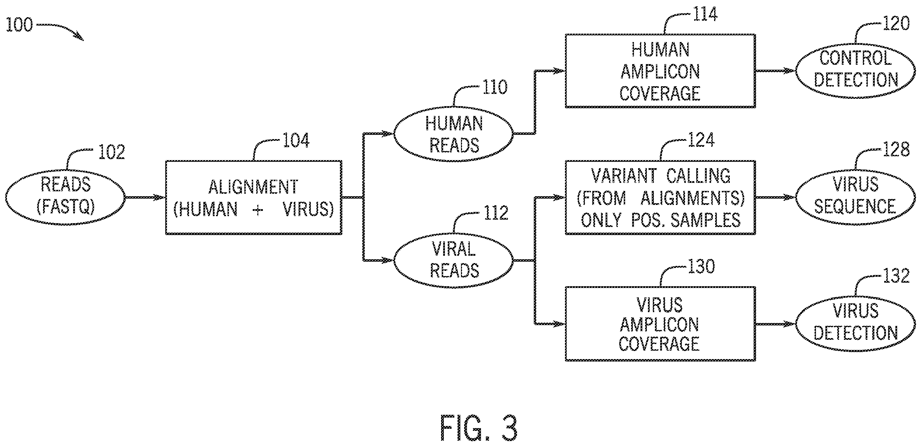

FIG. 3 shows an example method 100 that for viral pathogen detection in a human sample. The human sample sequence data 102 in the illustrated embodiment is provided as data in FASTQ format, which permits secondary analysis and alignment of the sequence reads to be performed for example using DRAGEN or another secondary analysis tool. Alignment 104 of the sequence reads can be performed using the k-mer aligner 36 (see FIG. 1) to identify exact matchers for k-mers of fixed size n using a set of reference k-mers based on the genome of the viral pathogen. The alignment 104 may also include identifying exact k-mer matches in the sequence data 102 for one or more human control amplicons (e.g., 2-15 amplicons) used as a measure of sample quality. In some embodiments, alignment 104 can be regular DRAGEN alignment to a reference genome including the virus, e.g., SARS-CoV2, and one or more human control amplicons.

The human reads 110 and the viral reads 112 are subjected to additional metrics as provided herein to assess sample quality based on human amplicon coverage 114 to generate a control detection output 120. The metrics also include virus amplicon coverage metrics 130 to provide a virus detection output 132. Positive samples, based on both the virus detection put and the control detection output 120, can be passed to variant calling 124 to generate a virus sequence output 128.

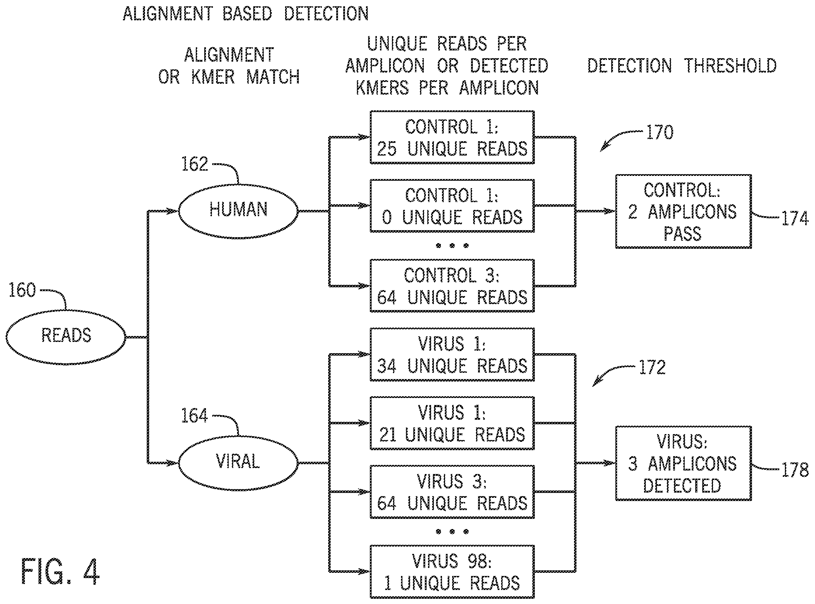

Once alignment/matching of the sequence reads using the k-mer aligner 36 has been performed, metrics related to the specified virus are interpreted and a determination is made on detection of the virus and an internal (human) control, as illustrated in FIG. 4. In some methods, the number of unique reads 160 mapping to the target region (or detected k-mers) of each amplicon may be counted.

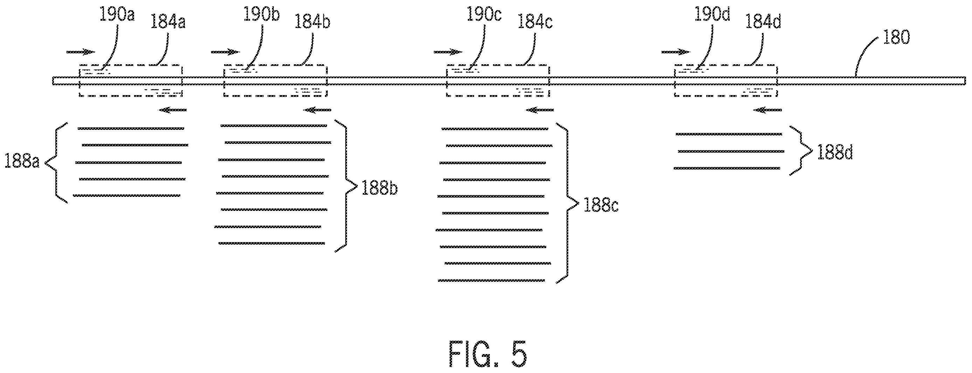

The 'target region', as illustrated in FIG. 5, can be in embodiments defined as the amplicon sequence 184, minus the primers and minus any overlap with another amplicon 184. This can be done either by a) aligning reads to the virus genome 180 and counting the number of (possibly de-duplicated) reads 188 mapping to the locations of each amplicon; or b) by counting the number of k-mers 190 from each amplicon sequence 184 that are observed in reads. The number of k-mers, or reads, are compared to a threshold of per-amplicon coverage to call each amplicon 184 either 'covered' or 'not covered'. If more than a second set threshold of virus amplicons 184 are covered, the call or viral detection output is that the virus is detected. The number of total amplicons depends on the assay used. In the example of FIG. 5, the amplicons 184 are nonoverlapping. However, it should be understood that more and overlapping amplicons 184 may be used to achieve up to whole genome coverage for the virus.

Returning to FIG. 4, after alignment and/or k-mer identification for human amplicons 162 and viral amplicons164, the coverage 170 of each individual human amplicon and the coverage 172 of each viral amplicon is counted. The read count (or count of k-mers detected) per amplicon is compared to a target threshold' to determine covered amplicons. The number of covered amplicons is then used to detect the virus 178 (with a positive detection result based on covered amplicons greater than or equal to a virus threshold) and the internal (human) control 174 (with a positive control detection result based on covered amplicons greater than or equal to a human control threshold). The threshold for detection of a positive amplicon and for number amplicons required to detect control and/or virus may vary. In some embodiments, the detection threshold may be as low as 2 amplicons, or may be higher, e.g., three, four, or more amplicons. In an embodiment, the threshold number of covered amplicons may be at least 1% at least 10%, or at least 50% of a total number of amplicons. In an embodiment, the threshold number of covered amplicons may in a range of 1-5% of a total number of amplicons of the assay. Because the detection is designed to provide fast results on real-time sequence data as additional sequence data is being generated from the sample, setting a percentage threshold permits the detection to be made based on any combination of positive amplicons. Thus, detection is independent of sample variation in location of sequenced clusters or other detection-specific variables that different from sample to sample.

FIG. 4 shows an example viral detection performed with a human control. For human control amplicons 170, control 1 had 25 unique reads and control 3 had 64 unique reads which passed the target threshold and these amplicons were determined to be covered amplicons. In the next step, 2 positive amplicons for the human control were compared to the human control threshold set equal to 2 or more, which resulted in a pass determination 174 for control detection threshold. Thus, the human control detection 174 included a two-step analysis of determining individual human amplicon coverage based on amplicon coverage thresholds and then assessing a number of amplicons that passed the coverage threshold. Likewise, the viral detection 178 included a first step in which the number of unique reads for each virus amplicon (e.g., Virus 1, Virus 2, Virus 3, etc.) was counted. Virus 1 had 34 unique reads, virus 2 had 21 unique reads, and virus amplicon 3 had 64 unique reads, and were all considered covered amplicons, but virus amplicon 98, which only had 1 unique read, was not considered a covered amplicon. In the next step, the 3 covered amplicons were compared to the viral threshold set equal to 3 or more which resulted in a virus detected result.

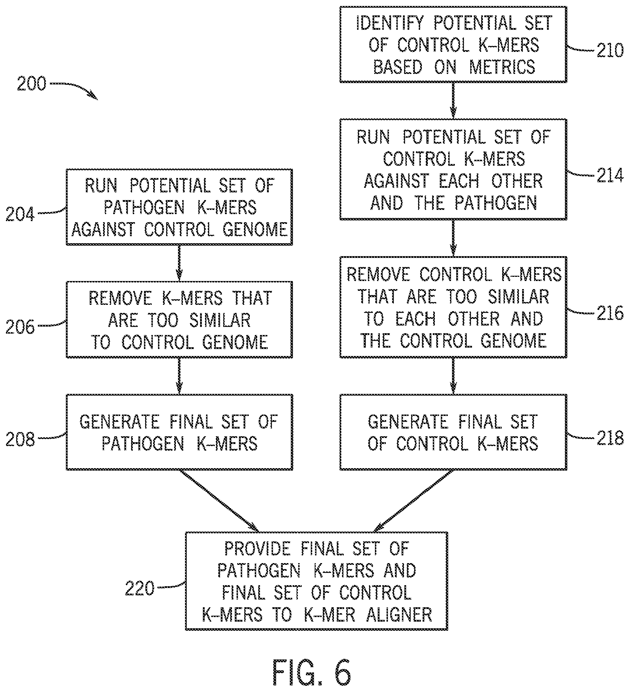

The disclosed techniques include quality and control parameters for establishing a set of reference and/or control k-mers used in a k-mer aligner (e.g., the k-mer aligner 36) for k-mer based alignment. FIG. 6 is a schematic illustration of a method 200 of generating a set of pathogen-specific k-mers and control k-mers for pathogen detection. A given pathogen genome includes a set of all potential k-mer of fixed size n, where n may be greater than 24 bases. However, certain of these k-mers may have exact matches within the control genome (e.g., the human genome). At block 204, potential pathogen k-mers are run against the control genome, and certain k-mers may be removed at block 206 to generate a final set of pathogen k-mers at block 208. In one example, k-mers in the potential set having exact matches against the control genome are removed. In another example, k-mers above a threshold similarity with the control genome are removed, e.g., a k-mer above a threshold similarity may include k-mers that have 1-3 bases (contiguous or non-contiguous) that are different from the control genome. For example, for a k-mer of a fixed size 32, a potential k-mer that has a 31/32 or 30/32 sequence match with the control genome is removed to account for potential base call errors that may yield detection false positives. Thus, the retained k-mers in the final set at block 208 may include k-mers with no eact match in the control genome and/or k-mers that have sufficient nonsimilarity to the control genome (e.g., differ by 1-3 bases within the k-mer).

A set of control k-mers can be selected from a pool of potential k-mers based on metrics at block 210. In an assay that sequences RNA in a human sample to detect the presence of an RNA virus, the human sample will also include human RNA, e.g., mRNA. Thus, a set of human control k-mers may be based on mRNA sequences that are likely to be always expressed in the sample tissue. The set of control k-mers may be selected to be smaller than the reference set, e.g., may include a smaller number of amplicons. The potential set of control k-mers is run against each other and, in embodiments, to the reference genome at block 214, and control k-mers that are exact matches or too similar to each other (e.g., having 1-3 bases that are different but otherwise having an exact match) and to the control genome are removed at step 216 to generate a final set of control k-mers at block 218. The final set of pathogen k-mers and the final set of control k-mers are provided to the k-mer aligner at block 220.

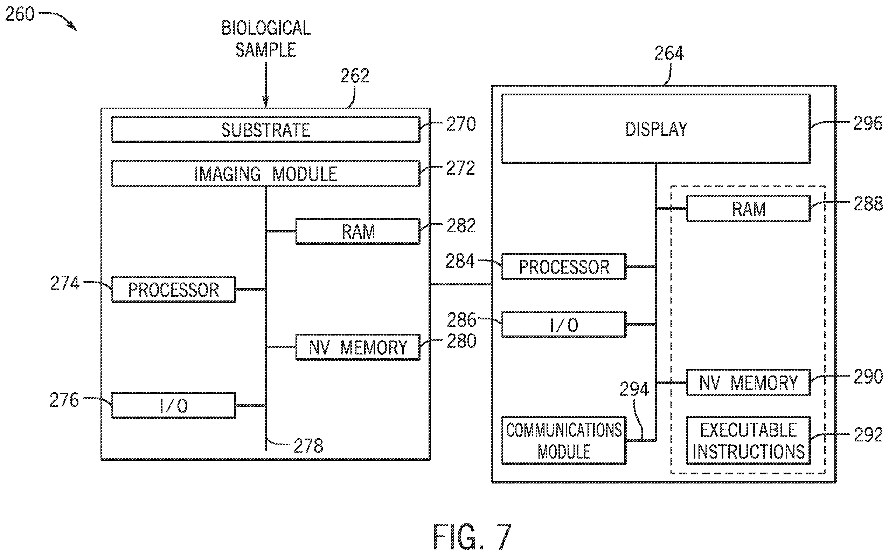

FIG. 7 is a schematic diagram of a sequencing device 260 that may be used in conjunction with the disclosed embodiments for acquiring sequence data from a sample as provided herein. The sequencing device 260 can conduct a sequencing run on a sample to acquire the sequence data. The sequencing device 260 may be implemented according to any sequencing technique, such as those incorporating sequencing-by-synthesis methods described in U.S. Patent Publication Nos. 2007/0166705; 2006/0188901; 2006/0240439; 2006/0281109; 2005/0100900; U.S. Pat. No. 7,057,026; WO 05/065814; WO 06/064199; WO 07/010,251. Alternatively, sequencing by ligation techniques may be used in the sequencing device 260. Such techniques use DNA ligase to incorporate oligonucleotides and identify the incorporation of such oligonucleotides and are described in U.S. Pat. No. 6,969,488; U.S. Pat. No. 6,172,218; and U.S. Pat. No. 6,306,597. Some embodiments can utilize nanopore sequencing, whereby sample nucleic acid strands, or nucleotides exonucleolytically removed from sample nucleic acids, pass through a nanopore. As the sample nucleic acids or nucleotides pass through the nanopore, each type of base can be identified by measuring fluctuations in the electrical conductance of the pore (U. S. Patent No. 7,001,792; Soni & Meller, Clin. Chem. 53, 1996-2001 (2007); Healy, Nanomed. 2, 459-481 (2007); and Cockroft, et al. J. Am. Chem. Soc. 130, 818-820 (2008)). Yet other embodiments include detection of a proton released upon incorporation of a nucleotide into an extension product. For example, sequencing based on detection of released protons can use an electrical detector and associated techniques that are commercially available from Ion Torrent (Guilford, CT, a Life Technologies subsidiary) or sequencing methods and systems described in US 2009/0026082 A1; US 2009/0127589 A1; US 2010/0137143 A1; or US 2010/0282617 A1.

Particular embodiments can utilize methods involving the real-time monitoring of DNA polymerase activity. Nucleotide incorporations can be detected through fluorescence resonance energy transfer (FRET) interactions between a fluorophore-bearing polymerase and γ-phosphate-labeled nucleotides, or with zeromode waveguides as described, for example, in Levene et al. Science 299, 682-686 (2003); Lundquist et al. Opt. Lett. 33, 1026-1028 (2008); Korlach et al. Proc. Natl. Acad. Sci. USA 105, 1176-1181 (2008). Other suitable alternative techniques include, for example, fluorescent in situ sequencing (FISSEQ), and Massively Parallel Signature Sequencing (MPSS). In particular embodiments, the sequencing device 260 may be an iSeq from Illumina (La Jolla, CA). In other embodiment, the sequencing device 260 may be configured to operate using a CMOS sensor with nanowells fabricated over photodiodes such that DNA deposition is aligned one-to-one with each photodiode.

In the depicted embodiment, the sequencing device 260 includes a separate sample substrate 262, e.g., a flow cell or sequencing cartridge, and an associated computer 264. However, as noted, these may be implemented as a single device. In the depicted embodiment, the biological sample may be loaded into substrate 262 that is imaged to generate sequence data. For example, reagents that interact with the biological sample fluoresce at particular wavelengths in response to an excitation beam generated by an imaging module 272 and thereby return radiation for imaging. For instance, the fluorescent components may be generated by fluorescently tagged nucleic acids that hybridize to complementary molecules of the components or to fluorescently tagged nucleotides that are incorporated into an oligonucleotide using a polymerase. As will be appreciated by those skilled in the art, the wavelength at which the dyes of the sample are excited and the wavelength at which they fluoresce will depend upon the absorption and emission spectra of the specific dyes. Such returned radiation may propagate back through the directing optics. This retrobeam may generally be directed toward detection optics of the imaging module 272, which may be a camera or other optical detector.

The imaging module detection optics may be based upon any suitable technology, and may be, for example, a charged coupled device (CCD) sensor that generates pixilated image data based upon photons impacting locations in the device. However, it will be understood that any of a variety of other detectors may also be used including, but not limited to, a detector array configured for time delay integration (TDI) operation, a complementary metal oxide semiconductor (CMOS) detector, an avalanche photodiode (APD) detector, a Geiger-mode photon counter, or any other suitable detector. TDI mode detection can be coupled with line scanning as described in U.S. Patent No. 7,329,860. Other useful detectors are described, for example, in the references provided previously herein in the context of various nucleic acid sequencing methodologies.

The imaging module 272 may be under processor control, e.g., via a processor 274, and may also include I/O controls 276, an internal bus 278, non-volatile memory 280, RAM 282 and any other memory structure such that the memory is capable of storing executable instructions, and other suitable hardware components that may be similar to those described with regard to FIG. 7. Further, the associated computer 264 may also include a processor 184, I/O controls 286, a communications module 294, and a memory architecture including RAM 288 and non-volatile memory 290, such that the memory architecture is capable of storing executable instructions 292. The hardware components may be linked by an internal bus 294, which may also link to the display 296. In embodiments in which the sequencing device 260 is implemented as an all-in-one device, certain redundant hardware elements may be eliminated.

The processor (e.g., the processor 274, 284) may be programmed to assign individual sequencing reads to a sample based on the associated index sequence or sequences according to the techniques provided herein. In particular embodiments, based on the image data acquired by the imaging module 272, the sequencing device 260 may be configured to generate sequencing data that includes sequence reads for individual clusters, with each sequence read being associated with a particular location on the substrate 270. Each sequence read may be from a fragment containing an insert. The sequencing data includes base calls for each base of a sequencing read. Further, based on the image data, even for sequencing reads that are performed in series, the individual reads may be linked to the same location via the image data and, therefore, to the same template strand. In this manner, index sequencing reads may be associated with a sequencing read of an insert sequence before being assigned to a sample of origin. The processor 274 may also be programmed to perform downstream analysis on the sequences for a particular sample subsequent to assignment of sequencing reads to the sample.

). In certain embodiments, the executable instructions 292 cause the processor to performs one of more actions of the methods disclosed herein. The processor (e.g., the processor 274, 284) may be a highly reconfigurable field-programmable gate array technology (FPGA). The processor (e.g., the processor 274, 284) may be programmed to receive user input for a particular analysis workflow to access a hash table including an appropriate set of reference k-mers and/or control k-mers stored in the memory (e.g., the memory 280, 290). In one example, the device 260 receives a user input selecting a run or panel of interest, and the k-mer aligner aligns streaming sequence to identify exact k-mer matches in the sequence data using a hash table associated with the user input. The memory may store multiple different sets of k-mers or different initialized hash tables that are specifically selected based on the user input. In an embodiment, the selection may also include a control k-mer selection. For example, the control k-mers may include human, mammalian, or other host organism control k-mers.

The disclosed techniques may be used to characterize a sample, e.g., a biological sample. The sample can be derived from any in vivo or in vitro source, including from one or multiple cells, tissues, organs, or organisms, whether living or dead, or from any biological or environmental source (e.g., water, air, soil). For example, in some embodiments, the sample nucleic acid comprises or consists of eukaryotic and/or prokaryotic dsDNA that originates or that is derived from humans, animals, plants, fungi, (e.g., molds or yeasts), bacteria, viruses, viroids, mycoplasma, or other microorganisms. In some embodiments, the sample nucleic acid comprises or consists of genomic DNA, subgenomic DNA, chromosomal DNA (e.g., from an isolated chromosome or a portion of a chromosome, e.g., from one or more genes or loci from a chromosome), mitochondrial DNA, chloroplast DNA, plasmid or other episomal-derived DNA (or recombinant DNA contained therein), or double-stranded cDNA made by reverse transcription of RNA using an RNA-dependent DNA polymerase or reverse transcriptase to generate first-strand cDNA and then extending a primer annealed to the first-strand cDNA to generate dsDNA. In some embodiments, the sample nucleic acid comprises multiple dsDNA molecules in or prepared from nucleic acid molecules (e.g., multiple dsDNA molecules in or prepared from genomic DNA or cDNA prepared from RNA in or from a biological (e.g., cell, tissue, organ, organism) or environmental (e.g., water, air, soil, saliva, sputum, urine, feces) source. In some embodiments, the sample nucleic acid is from an in vitro source. For example, in some embodiments, the sample nucleic acid comprises or consists of dsDNA that is prepared in vitro from single-stranded DNA (ssDNA) or from single-stranded or double-stranded RNA (e.g., using methods that are well-known in the art, such as primer extension using a suitable DNA-dependent and/or RNA-dependent DNA polymerase (reverse transcriptase). In some embodiments, the sample nucleic acid comprises or consists of dsDNA that is prepared from all or a portion of one or more double-stranded or single-stranded DNA or RNA molecules using any methods known in the art, including methods for: DNA or RNA amplification (e.g., PCR or reverse-transcriptase-PCR (RT-PCR), transcription-mediated amplification methods, with amplification of all or a portion of one or more nucleic acid molecules); molecular cloning of all or a portion of one or more nucleic acid molecules in a plasmid, fosmid, BAC or other vector that subsequently is replicated in a suitable host cell; or capture of one or more nucleic acid molecules by hybridization, such as by hybridization to DNA probes on an array or microarray.

The advantages of the disclosed techniques include suppression of noise (e.g. cross-contamination) that shows up as reads uniformly scattered through the virus genome, as opposed to real signal that clusters by amplicon. The technique is adaptable for different amplicons with different PCR performance by setting a variable per-amplicon threshold (higher to strongly amplified amplicons). The disclosed techniques have close correspondence to existing qPCR tests that also report a number of positive amplicons and are therefore output results easily translated for clinical use. The detection outputs, per sample may be reported out and or subject to downstream quality control.

In some embodiments, for any positive sample variant calling data may also be reported out. In some embodiments, a positive sample may be identified, and the techniques include providing notifications or recommendations for treatment based on the diagnosis of a positive sample. In an embodiment, a patient from whom the sample was taken is administered a treatment for the detected pathogen based on a diagnosis of pathogen detection or no pathogen detection according to the disclosed techniques and used as a point-of-care detection system. For example, if the detected pathogen is based on detection of the SARS-CoV-2 genome, a SARS-CoV-2 treatment is administered or a monitoring protocol is initiated. If no SARS-CoV-2 genome is detected, a SARS-CoV-2 vaccine may be administered based on the diagnosis of no active infection.

Additional advantages of the disclosed techniques include real-time quality metrics that are generated on-machine and variant detection. In the example of FIG. 7, the real-time quality metrics are generated on the sequencing device 260 and not as part of cloud-based secondary analysis. In a specific embodiment, sequence data may be analyzed based on the presence of and distribution of variants, which may include alternate alleles or single nucleotide polymorphism (SNP). The assay may include amplicon generation and/or targeted sequencing based on the desired variant or SNP analysis. For any particular detected allele, the distribution of the allele within the sequence reads can be assessed to yield a quality metric on-machine. The allele detection may be alignment-based or using k-mer matches as provided herein. The k-mer approach can be extended to detect known variants by including alternative allele versions of k-mers, one representing the reference, the other an alternate allele. The reference allele k-mers and alternative allele k-mers may include respective k-mer sets spanning the location of the variant sequence or sequences. With these modifications, an alignment free (and therefore fast) version of algorithms that can be executed while the sequencing device 260 is still generating data.

For a given variant and for a given individual sample, the allele distribution may be according to predictable levels. In one example, a particular germline variant allele, if present, is likely to be 50% distribution (with 50% of sequence reads at the location having one allele and the other 50% having the other allele) or 100% within the reads. Further, the case of no detected germline variant is likely to be 0% in the sequencing reads. Thus, a ratio of 1:1, 1:0 of variant vs. reference may be considered to be within an expected distribution for a detected germline variant. However, a distribution of 80%-20% or 95%-5% within reads for an individual sample is biologically unlikely and is therefore potentially a result of error or contamination. Thus, a ratio that deviates from a 1:1 ratio or a 1:0 ratio (e.g., within a 5%-10% tolerance to account for sequencing errors) is likely to be a sequencing artifact and/or based on sample contamination. Thus, the sequencing device 260 can assess a sample for germline variant allele distribution based on variant detection within the sequence reads.

For a given variant panel, e.g., a SNP panel, only a few of the variants may be matched for a particular sample. However, for variants that are detected and that deviate from expected allele distribution, the anomalous distribution can be a sign of sample contamination, patient identification or sample identification errors in assigning sample reads, or sample preparation issues. Thus, a sample that includes a variant with a distribution with an anomalous distribution or low frequency distribution (e.g., 95%-5%) can be flagged. The sequencing device 260, responsive to the flagging, can prove an error message (e.g. a displayed notification) on a graphical user interface in real time identifying the potentially contaminated sample. Accordingly, the disclosed techniques include real-time sample quality metrics for a sequencing device 260. Samples can be indicated as passing or failing depending on one or more assessed allele distributions. In an embodiment, failing for only one allele distribution is sufficient for flagging a sample. For k-mer based detection, the computationally-generated set of k-mers of variants or alternate alleles can be updated as new variants or strains are tracked.

Identification of a flagged or failing sample based on anomalous allele distribution can cause the sequencing device 260 to halt communication of the associated sequence data for the sample to cloud-based secondary analysis. Thus, for a multi-sample or multiplexed run, the sequencing device 260 can communicate only passing samples to the cloud for further analysis. If multiple samples all include the same anomalous allele distribution, the entire multiplexed run may be flagged as potentially contaminated.

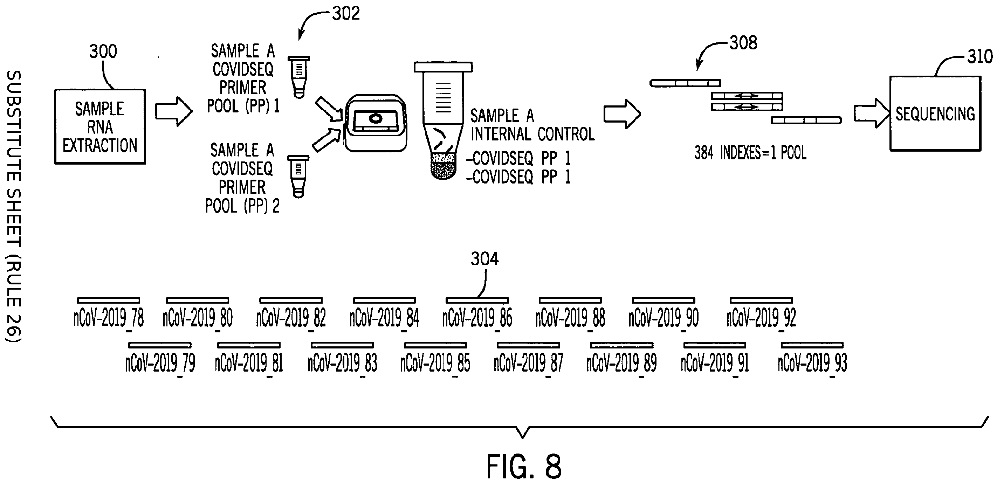

In embodiments, the disclosed techniques include improved the detection of variants that may be masked in sequence data based on primer design or position. For example, variants in regions of the genome that correspond to primer regions can be identified based on overlapping amplicon design, whereby the primer regions are covered by genomic reads from an overlapping amplicon. FIG. 8 shows an example workflow for sample preparation for pathogen detection that may be used in conjunction with the disclosed variant detection techniques. In the illustrated example, the sample is processed to extract RNA at block 300. RNA can be extracted from the sample such as a nasal pharyngeal swab.

The extracted RNA is converted to cDNA, and the cDNA is used to generate amplicons using an assay-specific primer set. For example, for COVIDSeq applications, the cDNA is split into two portions, and two different primer pools are used to generate different, overlapping, amplicons 304 between the two portions. Each sample is indexed, e.g., via tagmentation, at step 308, and sequenced at step 310.

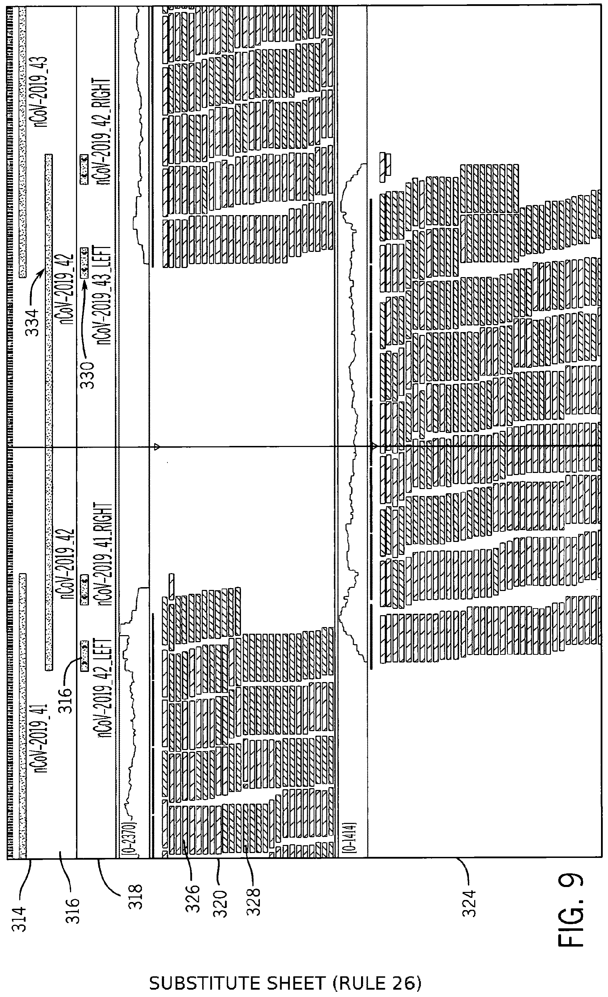

FIG. 9 shows amplicon sequencing results for the workflow of FIG. 8 showing coverage of overlapping amplicons 314 generated from the first primer pool and amplicons 316 generated from the second primer pool for sequence reads that are indexed together, e.g., that are indexed as originating from a same sample. Pool 1 reads 320 and pool 2 reads 324 include overlapping portions at their edges in a primer region. Within the pool 1 reads 320 and the pool 2 reads 324 are forward reads 326 and reverse reads 328. Fragmentation after PCR has the effect of partially depleting primers, which leads to edge effects such that reads are clustered towards the primer-side in both the forward and reverse direction. The sequence reads in an overlapping region include a heterogeneous mixture of genomic reads, with the variant, and primer reads, with the reference sequence.

However, the clustering of primer reads towards the ends of the amplicons may lower observed alternative allele fractions, because the primer reads represent an inflated portion of the mix due to edge effects. For example, the forward primer 330 overlaps with an internal region of another amplicon 334. The pool 1 reads 320 are all forward reads 326 that originate from the primer 330. The pool 2 reads 324 include both forward reads 326 and reverse reads 328. Reads 320 in pool 1 that originate from the primer 330 would be an exact primer match and would, therefore, not include any variants present in the region of genome covered by the primer 330.

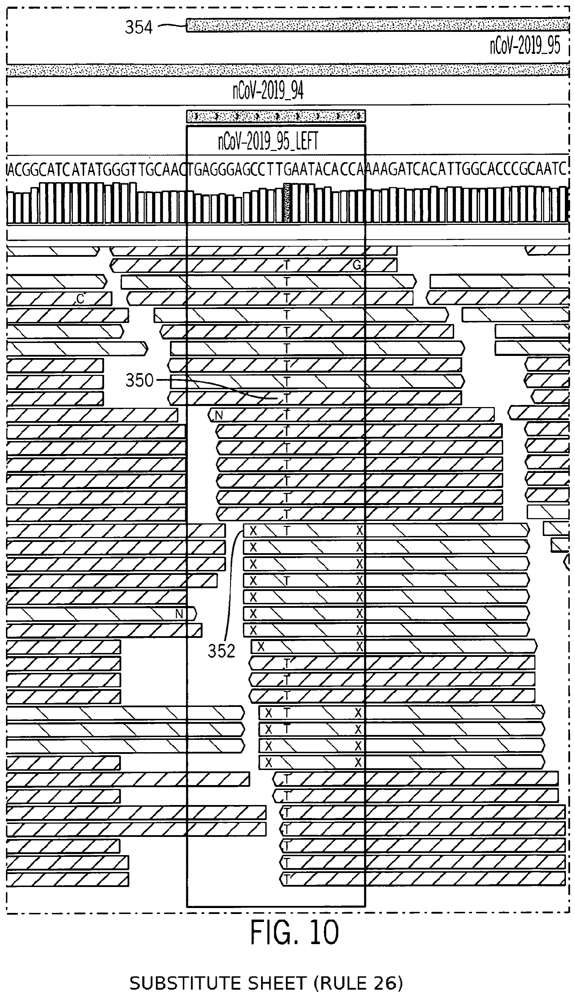

To improve the sensitivity for variant detection, the disclosed techniques include a primer trimming step that hard clips, masks, or removes primer sequences from reads. The filter trims reads 1) starting in primer regions and 2) matching primer orientation. That is, any sequence read with a first nucleotide starting in a region covered by a primer and that is a forward read in a forward primer direction or a reverse read in a reverse primer direction is trimmed. However, coverage in primer region remains from overlapping amplicon spanning reads and any opposite strand (complementary) reverse reads. FIG. 10 shows an example of trimmed reads in a region covered by a primer of the reaction. The read mixture includes untrimmed or retained reads 350 and trimmed reads 352. The reads 352 are trimmed only in the sequences corresponding to the primer region 354, indicated by starting and ending Xs, and only forward reads are trimmed. As illustrated, the retained reads 350 predominantly include a G to T variant. The T variant is not observed in most of the trimmed reads. In one example, a variant is called based on a threshold percentage (e.g., at least 50%) of untrimmed reads 350 including the variant.

Table 1 shows an example of improved detection of the single G to T variant. After filter trimming, the remaining allele fractions showed convergence towards almost 100% allele fraction, which would be an expected biological distribution, in the remaining reads.

**Table 1: Effect of primer trimming on detected allele fractions G to T**

| Sample | Unfiltered AF [%] | Filtered AF [%] | Fraction Filtered [%] |
|---|---|---|---|
| 1 | 93.2 | 99.8 | 12 |
| 2 | 95.5 | 99.6 | 10 |
| 3 | 98.3 | 99.8 | 7 |
| 4 | 94.3 | 97.6 | 12 |
| 5 | 85.9 | 99.9 | 26 |
| 6 | 96.1 | 99.8 | 10 |
| 7 | 95.8 | 99.9 | 10 |
| 8 | 97.7 | 99.9 | 9 |
| 9 | 95.9 | 99.9 | 9 |
| 10 | 98.5 | 99.9 | 7 |
| 11 | 82.4 | 97.6 | 28 |
| 12 | 87.9 | 99.5 | 18 |
| 13 | 99.7 | 99.9 | 7 |

While the depicted embodiment shows trimming for a single primer, the primer trimming can be used to cover all primers in the reaction, both forward and reverse, to improve variant identification in any region covered by a primer. For whole genome sequencing of pathogens in which several, e.g., 50 or more, primer pairs are used, the primer trimming may significantly improve variant detection.

This written description uses examples in embodiments of the disclosure, including the best mode, and also to enable any person skilled in the art to practice the disclosed embodiments, including making and using any devices or systems and performing any incorporated methods. The patentable scope of the disclosure is defined by the claims.

## Claims

1. A real-time quality control method, comprising:
generating sequence data from a biological sample using a sequencing device conducting a sequencing run;
identifying k-mers in the sequence data that have an exact match in a hash table that is initialized with a set of k-mers comprising reference allele k-mers and alternative allele k-mers of the reference allele;
determining a distribution of the reference allele and the alternative allele in the sequence data based on a count of the exact matches of the reference allele k-mers relative to the alternative allele k-mers; and
generating a passing or failing quality metric on the sequencing device for the biological sample based on the distribution and during the sequencing run of the biological sample.

2. The method of claim 1, comprising flagging the biological sample as contaminated based on the failing quality metric.

3. The method of claim 2, wherein the alternative allele is present in 5% or less of sequence reads of the sequence data in the contaminated sample.

4. The method of claim 1, comprising generating the passing quality metric based on a ratio of the reference allele to the alternative allele in the sequence data being within a range.

5. The method of claim 1, wherein the alternative allele comprises a previously characterized single nucleotide polymorphism.

6. The method of claim 4, wherein the passing quality metric is based on the distribution being with 5-10% tolerance of a 1:1 ratio reference allele: alternative allele or a 1:0 ratio reference allele: alternative allele of the count of the exact matches of the reference allele k-mers relative to the alternative allele k-mers.

7. A sequencing device, comprising:
a substrate having loaded thereon a sequencing library prepared from a sample;
a computer programmed to:
cause the sequencing device to conduct a sequencing run to generate sequence data from sequencing library;
identify k-mers in the sequence data that have an exact match in a hash table that is initialized with a set of k-mers comprising reference allele k-mers and alternative allele k-mers of the reference allele;
determine a distribution of the reference allele and the alternative allele in the sequence data based on a count of the exact matches of the reference allele k-mers relative to the alternative allele k-mers; and
generate a failing quality metric on the sequencing device for the biological sample based on the distribution being indicative of error or contamination during the sequencing run.

8. The sequencing device of claim 7, comprising a display that displays the quality metric.

9. The sequencing device of claim 7, comprising communication circuitry that communicates the generated sequence data to a cloud computing environment based on the quality metric of the biological sample being associated with passing.

10. The sequencing device of claim 9, further comprising generating a passing quality metric of the biological sample associated with a ratio of the reference allele and the alternative allele being within a range.

11. The sequencing device of claim 7, comprising communication circuitry that halts communication of the generated sequence data to a cloud computing environment based on the quality metric of the biological sample being associated with failing.

12. The sequence device of claim 7, wherein the passing quality metric is based on the distribution being within 5-10% tolerance of a 1:1 ratio reference allele: alternative allele or a 1:0 ratio reference allele: alternative allele of the count of the exact matches of the reference allele k-mers relative to the alternative allele k-mers.

## Patentansprüche

1. Echtzeit-Qualitätskontrollverfahren, das Folgendes umfasst:
Erzeugen von Sequenzdaten aus einer biologischen Probe unter Verwendung einer Sequenzierungsvorrichtung, die einen Sequenzierungsdurchlauf durchführt;
Identifizieren von K-Meren in den Sequenzdaten, die eine genaue Übereinstimmung in einer Hash-Tabelle aufweisen, die mit einem Satz von K-Meren initialisiert wird, der Referenzallel-K-Mere und Alternativallel-K-Mere des Referenzallels umfasst;
Bestimmen einer Verteilung des Referenzallels und des Alternativallels in den Sequenzdaten basierend auf einer Anzahl der genauen Übereinstimmungen der Referenzallel-K-Mere relativ zu den Alternativallel-K-Meren; und
Erzeugen einer Bestanden- oder Nicht-Bestanden-Qualitätsmetrik auf der Sequenzierungsvorrichtung für die biologische Probe, basierend auf der Verteilung und während des Sequenzierungsdurchlaufs der biologischen Probe.

2. Verfahren nach Anspruch 1, das Markieren der biologischen Probe als kontaminiert basierend auf der Nicht-Bestanden-Qualitätsmetrik umfasst.

3. Verfahren nach Anspruch 2, wobei das Alternativallel in 5% oder weniger Sequenzablesungen der Sequenzdaten in der kontaminierten Probe vorhanden ist.

4. Verfahren nach Anspruch 1, das Erzeugen der Bestanden-Qualitätsmetrik basierend darauf umfasst, dass ein Verhältnis des Referenzallels zum Alternativallel in den Sequenzdaten innerhalb eines Bereichs liegt.

5. Verfahren nach Anspruch 1, wobei das Alternativallel einen zuvor charakterisierten Einzelnukleotid-Polymorphismus umfasst.

6. Verfahren nach Anspruch 4, wobei die Bestanden-Qualitätsmetrik darauf basiert, dass die Verteilung eine Toleranz von 5-10 % eines 1:1 Verhältnisses von Referenzallel: Alternativallel oder eines 1:0 Verhältnisses von Referenzallel: Alternativallel der Anzahl der genauen Übereinstimmungen der Referenzallel-K-Mere relativ zu den Alternativallel-K-Meren aufweist.

7. Sequenzierungsvorrichtung, umfassend:
ein Substrat, das eine aus einer Probe erstellte Sequenzierungsbibliothek darauf geladen aufweist;
einen Computer, der für Folgendes programmiert ist:
Veranlassen der Sequenzierungsvorrichtung, einen Sequenzierungsdurchlauf durchzuführen, um Sequenzdaten aus der Sequenzierungsbibliothek zu erzeugen;
Identifizieren von K-Meren in den Sequenzdaten, die eine genaue Übereinstimmung in einer Hash-Tabelle aufweisen, die mit einem Satz von K-Meren initialisiert wird, der Referenzallel-K-Mere und Alternativallel-K-Mere des Referenzallels umfasst;
Bestimmen einer Verteilung des Referenzallels und des Alternativallels in den Sequenzdaten basierend auf einer Anzahl der genauen Übereinstimmungen der Referenzallel-K-Mere relativ zu den Alternativallel-K-Meren; und
Erzeugen einer Nicht-Bestanden-Qualitätsmetrik auf der Sequenzierungsvorrichtung für die biologische Probe, basierend darauf, dass die Verteilung einen Fehler oder eine Kontamination während des Sequenzierungsdurchlaufs angibt.

8. Sequenzierungsvorrichtung nach Anspruch 7, die eine Anzeige umfasst, die die Qualitätsmetrik anzeigt.

9. Sequenzierungsvorrichtung nach Anspruch 7, die eine Kommunikationsschaltung umfasst, die die erzeugten Sequenzdaten basierend darauf an eine Cloud-Rechenumgebung kommuniziert, dass die Qualitätsmetrik der biologischen Probe mit einem Bestehen verknüpft ist.

10. Sequenzierungsvorrichtung nach Anspruch 9, die weiter Erzeugen einer Bestanden-Qualitätsmetrik der biologischen Probe umfasst, die damit verknüpft ist, dass ein Verhältnis des Referenzallels und des Alternativallels innerhalb eines Bereichs liegt.

11. Sequenzierungsvorrichtung nach Anspruch 7, die eine Kommunikationsschaltung umfasst, die die Kommunikation der erzeugten Sequenzdaten basierend darauf stoppt, dass die Qualitätsmetrik der biologischen Probe mit einem Nicht-Bestehen verknüpft ist.

12. Sequenzvorrichtung nach Anspruch 7, wobei die Bestanden-Qualitätsmetrik darauf basiert, dass die Verteilung innerhalb einer Toleranz von 5-10 % eines 1:1 Verhältnisses von Referenzallel: Alternativallel oder eines 1:0 Verhältnisses von Referenzallel: Alternativallel der Anzahl der genauen Übereinstimmungen der Referenzallel-K-Mere relativ zu den Alternativallel-K-Meren liegt.

## Revendications

1. Procédé de contrôle qualité en temps réel comprenant :
la génération de données de séquence à partir d'un échantillon biologique à l'aide d'un dispositif de séquençage effectuant une exécution de séquençage ;
l'identification de k-mers dans les données de séquence qui ont une correspondance exacte dans une table de hachage qui est initialisée avec un ensemble de k-mers comprenant des k-mers d'allèle de référence et des k-mers d'allèle alternatif de l'allèle de référence ;
la détermination d'une distribution de l'allèle de référence et de l'allèle alternatif dans les données de séquence sur la base d'un décompte des correspondances exactes des k-mers de l'allèle de référence par rapport aux k-mers de l'allèle alternatif ; et
la génération d'une mesure de qualité de réussite ou d'échec sur le dispositif de séquençage pour l'échantillon biologique en fonction de la distribution et pendant l'exécution du séquençage de l'échantillon biologique.

2. Procédé selon la revendication 1, comprenant le marquage de l'échantillon biologique comme contaminé sur la base de la mesure de qualité d'échec.

3. Procédé selon la revendication 2, dans lequel l'allèle alternatif est présent dans 5 % ou moins de lectures de séquence des données de séquence dans l'échantillon contaminé.

4. Procédé selon la revendication 1, comprenant la génération de la mesure de qualité de réussite sur la base d'un rapport entre l'allèle de référence et l'allèle alternatif dans les données de séquence se situant dans une plage.

5. Procédé selon la revendication 1, dans lequel l'allèle alternatif comprend un polymorphisme nucléotidique unique précédemment caractérisé.

6. Procédé selon la revendication 4, dans lequel la mesure de qualité de réussite est basée sur la distribution ayant une tolérance de 5 à 10 % d'un rapport 1:1 d'allèle de référence : allèle alternatif ou d'un rapport 1:0 d'allèle de référence : allèle alternatif du décompte de correspondances exactes des k-mers de l'allèle de référence par rapport aux k-mers de l'allèle alternatif.

7. Dispositif de séquençage comprenant :
un substrat sur lequel est chargée une bibliothèque de séquençage préparée à partir d'un échantillon ;
un ordinateur programmé pour :
amener le dispositif de séquençage à effectuer une exécution de séquençage pour générer des données de séquence à partir de la bibliothèque de séquençage ;
identifier les k-mers dans les données de séquence qui ont une correspondance exacte dans une table de hachage qui est initialisée avec un ensemble de k-mers comprenant des k-mers d'allèle de référence et des k-mers d'allèle alternatif de l'allèle de référence ;
déterminer une distribution de l'allèle de référence et de l'allèle alternatif dans les données de séquence en fonction d'un décompte des correspondances exactes des k-mers de l'allèle de référence par rapport aux k-mers de l'allèle alternatif ; et
générer une mesure de qualité d'échec sur le dispositif de séquençage pour l'échantillon biologique en fonction de la distribution indiquant une erreur ou une contamination pendant l'exécution du séquençage.

8. Dispositif de séquençage selon la revendication 7, comprenant un affichage qui affiche la mesure de qualité.

9. Dispositif de séquençage selon la revendication 7, comprenant un circuit de communication qui communique les données de séquence générées à un environnement informatique en nuage sur la base de la mesure de qualité de l'échantillon biologique associé à la réussite.

10. Dispositif de séquençage selon la revendication 9, comprenant en outre la génération d'une mesure de qualité de réussite de l'échantillon biologique associée à un rapport de l'allèle de référence et de l'allèle alternatif se situant dans une plage.

11. Dispositif de séquençage selon la revendication 7, comprenant un circuit de communication qui arrête la communication des données de séquence générées vers un environnement informatique en nuage en fonction de la mesure de qualité de l'échantillon biologique associée à un échec.

12. Dispositif de séquençage selon la revendication 7, dans lequel la mesure de qualité de réussite est basée sur la distribution se situant dans une tolérance de 5 à 10 % d'un rapport 1:1 d'allèle de référence : allèle alternatif ou d'un rapport 1:0 d'allèle de référence : allèle alternatif du décompte de correspondances exactes des k-mers de l'allèle de référence par rapport aux k-mers de l'allèle alternatif.
